# Europäisches Patentamt

# European Patent Office

# Office européen des brevets

(19)

(11) Veröffentlichungsnummer : **0 014 956**
**B1**

(12) **EUROPÄISCHE PATENTSCHRIFT**

(45) Veröffentlichungstag der Patentschrift :
01.09.82

(51) Int. Cl.³ : **G 01 N 33/52, G 01 N 31/08**

(21) Anmeldenummer : 80100733.7

(22) Anmeldetag : 14.02.80

(54) **Mikrochromatographisches System für Urinproben zur Kontrolle der Arzneimitteleinnahme.**

(30) Priorität : **20.02.79 DE 7904648 U**

(43) Veröffentlichungstag der Anmeldung :
**03.09.80 (Patentblatt 80/18)**

(45) Bekanntmachung des Hinweises auf die Patenterteilung : **01.09.82 Patentblatt 82/35**

(84) Benannte Vertragsstaaten :
**AT BE CH DE FR GB IT LU NL SE**

(56) Entgegenhaltungen :
**DE A 2 601 794**
**DE A 2 711 201**
**FR A 2 356 944**
**US A 4 131 544**

(73) Patentinhaber : **HOECHST AKTIENGESELLSCHAFT**
**Postfach 80 03 20**
**D-6230 Frankfurt/Main 80 (DE)**

(72) Erfinder : **Uhlein, Michael, Dr.**
**Königsberger Strasse 52**
**D-6239 Kriftel (DE)**

Imprimerie Jouve, 18, rue St-Denis, 75001 Paris, France

EP 0 014 956 B1

# 0 014 956

Mikrochromatographisches System für Urinproben zur Kontrolle der Arzneimitteleinnahme

Die Erfindung betrifft ein mikrochromatographisches System für Urinproben zur Kontrolle der Arzneimitteleinnahme.

Es ist bekannt, daß es immer wieder Patienten gibt, die ein ihnen verordnetes Arzneimittel nicht oder nicht regelmäßig einnehmen. Solange dem Arzt dieser Umstand nicht bekannt ist, führt er den naturgemäß ausbleibenden Therapieerfolg auf die Unwirksamkeit des Arzneimittels zurück. Er wird dann in Zukunft bei ähnlichen Erkrankungen mit der Verabreichung des angeblich unwirksamen Arzneimittels vorsichtiger sein oder unter Umständen hierauf ganz verzichten. Darüberhinaus wird ihm der Mißerfolg seiner Therapie Veranlassung sein, seine an sich richtige Diagnose zu überprüfen, was ihn möglicherweise zu einer falschen Beurteilung der Erkrankung führt. Für den Arzt ist es deshalb sehr wichtig, kontrollieren zu können, ob das Ausbleiben des Therapieerfolges auf die Auswahl eines ungeeigneten Arzneimittels zurückzuführen ist oder ob die angebliche Umwirksamkeit nur dadurch zu erklären ist, daß der Patient den Fehlschlag durch eine Mißachtung der Einnahmevorschrift des Arzneimittels selbst verursacht hat.

Erfahrungsgemäß ist eine derartige Kontrolle dann mit großer Sicherheit durchfürbar, wenn

a) das Präparat überwiegend renal in einheitlicher Form (Originalsubstanz oder Metabolit) ausgeschieden wird und

b) die renale Ausscheidung innerhalb von 24 Stunden nach der Arzneimitteleinahme praktisch beendet ist.

Zweckmäßigerweise sollte diese Kontrolle durch dünnschichtchromatographischen Nachweis von Originalsubstanz oder Metabolit im Urin erfolgen. In der ärztlichen Praxis haben dünnschichtchromatographische Kontrollen der Arzneimitteleinnahme trotzdem noch keine Bedeutung erlangt. Das liegt daran, daß die bisher bekannten Nachweismethoden zu teure Apparate und eine chemischanalytische Vorbildung erfordern, die in der ärztlichen Praxis in der Regel nicht vorhanden sind.

Das Bedürfnis, einfach zu handhabende chromatographische Methoden für die ärztliche Praxis zur Verfügung zu haben, ist auch aus der deutschen Offenlegungsschrift 26 01 794 zu erkennen. Dort ist ein Test zum Nachweis von Okkultem Blut beschrieben, bei dem ein mit Nachweischemikalien getränkter, saugfähiger Träger an seinem einen Endemit den zu untersuchenden Körperausscheidungen in Berührung gebracht und dann in einem Röhrchen aus durchsichtigem Material chromatographiert wird. Bei Anwesenheit von okkultem Blut bildet sich ein Farbstoff, der in dem saugfähigen Träger nach oben wandert und dann auch von wenig geschulten Personen gut erkannt werden kann. Über die Verwendung zweier unterschiedlicher und von Nachweischemikalien freier Sorptionsmaterialien zur Beschichtung des Trägers und die damit erzielbaren analytischen Vorteile ist dort jedoch nichts berichtet.

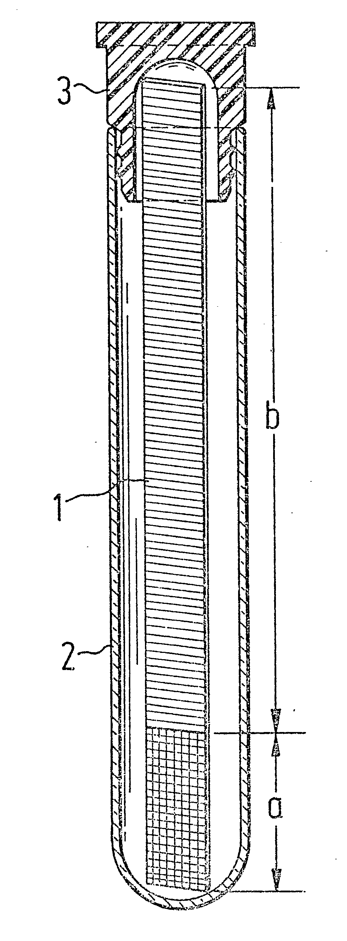

Deshalb ist das in der Abbildung dargestellte mikrochromatographische System für Urinproben zur Kontrolle der Arzneimitteleinnahme, das aus einem als Entwicklungskammer dienenden, aus durchsichtigem Material hergestellten Reagenzglas (2), das mit einem Stopfen (3) verschlossen werden kann, und einem in das Reagenzglas passenden Teststreifen besteht, erfindungsgemäß dadurch gekennzeichnet, daß als Teststreifen eine streifenförmige Dünnschichtplatte oder -folie (1) eingesetzt wird, die im unteren Viertel (Zone a) mit Diatomeenerde oder einem extrem weitporigen Silikagel mit geringer spezifischer Oberfläche und im übrigen (Zone b) mit einem engporigen Silikagel hoher spezifischer Oberfläche beschichtet ist, wie es für die Dünnschichtchromatographie üblicherweise verwendet wird.

Dieses dünnschichtchromatographische System ist so einfach und zweckmäßig gestaltet, daß es damit möglich ist, ohne chromatographische Ausrüstung und spezielle Reagenzien unter Verwendung eines mitgelieferten Laufmittelgemisches (0,5-1 ml) einen sicheren Nachweis des Arzneimittels oder seines Metaboliten im Urin durchzuführen. Das System ist für den einmaligen Gebrauch bestimmt.

Der Teststreifen ist zweckmäßigerweise eine Glasplatte oder Folie, die z.B. die Dimensionen 100 × 8 mm aufweisen kann. Er ist einseitig wie eine Dünnschichtplatte beschichtet.

Die im unteren Viertel (Zone a) aufgebrachte Schicht aus Diatomeenerde oder einem extrem weitporigen Silikagel hat eine spezifische Oberfläche unter 1 m²/g. Die sich daran unmittelbare anschließende Trennzone (Zone b) besteht aus einem engporigen Silikagel hoher spezifischer Oberfläche, wie es für die Dünnschichtchromatographie üblich ist.

Die Entwicklungskammer besteht vorzugsweise aus einem Polypropylen-Reagenzglas von ca. 15 mm Innendurchmesser und etwa 95 mm Höhe und einem Stopfen. Ihre Dimension ist damit so gestaltet, daß für die dünnschichtchromatographische Trennung nur 0,5 bis 1 ml Laufmittel benötigt werden. Eine derartig kleine Menge des Laufmittels kann — in der dem jeweiligen Verwendungszweck optimal angepaßten Zusammensetzung — in einer Braunglas-Schmelzampulle oder einem Glasgefäß mit abreißbarem Kapselverschluß mitgeliefert werden. Eine gute Reproduzierbarkeit der Trennung und damit des Arzneimittel-Nachweises wird erfahrungsgemäß durch Sättigung des Dampfraumes mit Laufmittel erreicht. Dies ist beim erfindungsgemäßen mikrochromatographischen System besonders einfach durch Schütteln des Laufmittels in der Entwicklungskammer vor Eintauchen des Teststreifens möglich.

Das Nachweisverfahren wird zweckmäßigerweise folgendermaßen durchgeführt :

Ca. 1 ml Urin wird in einem Reagenzglas vorgelegt. Durch Eintauchen der Konzentrierungszone für 10 bis 20 Sek. wird der Teststreifen mit Harn benetzt. Der anschließend getrocknete Teststreifen wird solange in die mit dem Laufmittel gefüllte Entwicklungskammer gestellt, bis die Fließgrenze fast das obere Ende des Teststreifens erreicht hat. Der wiederum getrocknete Teststreifen wird im UV-Licht auf das Vorhandensein charakteristischer Flecke in der Trennschicht untersucht. Die Beurteilung kann durch die Benutzung einer Schablone erleichert werden.

Das beschriebene mikrochromatographische System vereinigt alle Vorteile einer einfachen und sicheren dünnschichtchromatographischen Methode in sich. Es erfüllt ein schon lange bestehendes Bedürfnis, eine Methode zur Verfügung zu stellen, mit der nachgeprüft werden kann, ob der Patient das verordnete Arzneimittel tatsächlich eingenommen hat.

Die folgenden Beispiele zeigen die breite Anwendbarkeit des Verfahrens :

## Beispiel 1

a) Vier verschiedenen Patienten wurden 25 mg, 50 mg, 100 mg und 150 mg Nomifensin als Einmaldosis um 17.00 Uhr verabreicht. Am nächsten Morgen wurde der Urin gesammelt, jeweils 1 ml in ein Probenglas gefüllt und dann der Teststreifen mit der Konzentrierungsschicht nach unten eingestellt. Nach jeweils 15 Sek. wurde der Teststreifen herausgenommen, getrocknet und in ein anderes Probenglas gestellt, in dem sich 1 ml Fließmittel, bestehend aus 99,5 % Tetrahydrofuran und 0,5 % wässrigem Ammoniak befand. Das Probenglas wurde verschlossen und nach 15 Minuten der Teststreifen wieder entnommen. Nach Trocknung wurde der Teststreifen wieder entnommen. Nach Trocknung wurde der Teststreifen 15 Min. mit UV-Licht (254 μm) bestrahlt und dann unter dem UV-Licht zwischen 50 und 65 mm von unteren Rand des Teststreifens entfernt ein weiß bis hellgelb fluoreszierender Fleck nachgewiesen. Dieser Fleck ist charakteristisch für Nomifensin. Fluoreszierende Flecke in der Konzentrierungsschicht und am oberen Ende der Trennschicht treten immer auf und sind ohne Bedeutung.

b) In einem zweiten Versuch nahm je ein Patient Clomifensin (Mischung aus Clobazam und Nomifensin im Verhältnis 3 : 10), wobei der erste Patient 32,5 mg, der zweite 65 mg der Mischung und der dritte ein Placebo erhielt. Der Urin wurde in den Perioden 0-2 Stunden, 2-4 Stunden, 4-6 Stunden und 6-8 Stunden nach der Verabreichung gesammelt. Dabei wurden folgende Ergebnisse erzielt :

| Dosis | 0-2 h | 2-4 h | 4-6 h | 6-8 h |
|---|---|---|---|---|
| 32,5 mg | + | ++ | ++ | + |
| 65 mg | ++ | ++ | ++ | + |
| Placebo | - | - | - | - |

## Beispiel 2

In einem weiteren Versuch wurde einem Patienten 150 mg Carbocromen verabreicht. Der Urin wurde nach 0-1 Stunde, 1-2 Stunden, 2-4 Stunden, 4-6 Stunden, 6-8 Stunden, 8-10 Stunden und 24-26 Stunden nach Verabreichung gesammelt. Zum Nachweis wurde in gleicher Weise wie bei Beispiel 1 verfahren, nur wurde als Fließmittel die sich aus zwei Volumenteilen Chloroform, 3 Volumenteilen Isopropanol, einem Volumenteil Eisessig und einem Volumenteil bi-destilliertem Wasser in einem Scheidetrichter als untere Phase abscheidende Flüssigkeit verwendet. Der Teststreifen zeigte im Bereich zwischen 38 und 42 mm vom unteren Rand des Teststreifens einen weiß-fluoreszierenden Fleck, der für die aus Carbocromen entstehende Carbocromensäure charakteristisch ist. Folgendes Ergebnis wurde erhalten :

| Zeit | Ergebnis |
|---|---|
| 0 - 1 h | - |
| 1 - 2 h | + |
| 2 - 4 h | + |
| 4 - 6 h | + |
| 6 - 8 h | + |
| 8 - 10 h | (+) |
| 24 - 26 h | - |

3

**Ansprüche**

1. Mikrochromatographisches System für Urinproben zur Kontrolle der Arzneimitteleinnahme bestehend aus einem als Entwicklungskammer dienenden, aus durchsichtigem Material hergestellten Reagenzglas (2), das mit einem Stopfen (3) verschlossen werden kann, und einem in das Reagenzglas passenden Teststreifen, dadurch gekennzeichnet, daß als Teststreifen eine streifenförmige Dünnschichtplatte oder -folie (1) eingesetzt wird, die im unteren Viertel (Zone a) mit Diatomeenerde oder einem extrem weitporigen Silikagel mit geringer spezifischer Oberfläche und im übrigen (Zone b) mit einem engporigen Silikagel hoher spezifischer Oberfläche, wie es für die Dünnschichtchromatographie üblicherweise verwendet wird, beschichtet ist.

2. Mikrochromatographisches System gemäß Anspruch 1, dadurch gekennzeichnet, daß das als Entwicklungskammer dienende Reagenzglas (2) aus Polypropylen besteht.

**Claims**

1. Microchromatographic system for urine samples in order to control the taking of a medicine, which consists of a test tube (2) made of a transparent material and serving as developing chamber, which can be closed by a stopper (3), and a test strip fitting into the test tube, characterized by using as the test strip a thin-layer plate or foil in the form of a strip (1) which is coated in its lower quarter (zone a) with kieselguhr or a extremely wide-porous silicagel with low specific surface, the remainder (zone b) being covered with a narrow-porous silicagel of high specific surface as it is generally used in thin-layer chromatography.

2. Microchromatographic system according to claim 1, characterized in that the test tube (2) serving as developing chamber consists of polypropylene.

**Revendications**

1. Système microchromatographique de contrôle de prise de médicaments utilisant des échantillons d'urine, constitué d'un tube à essais (2) fabriqué en un matériau transparent, servant de chambre de développement qui peut être fermé avec un bouchon (3), et d'une bande d'essai pouvant entrer dans le tube à essais, caractérisé en ce que comme bande d'essai on utilise une plaque ou une feuille (1) pour couche mince qui est recouverte sur son quart inférieur (zone a) de terre de diatomées ou d'un gel de silice à pores extrêmement larges ayant une faible surface spécifique et sur le reste (zone b) d'un gel de silice à pores étroits d'une grande surface spécifique, comme on en utilise usuellement pour la chromatographie en couche mince.

2. Système microchromatographique selon la revendication 1, caractérisé en ce que le tube à essais (2) servant de chambre de développement est en polypropylène.